# EUROPEAN PATENT SPECIFICATION

(11) **EP 2 464 382 B1**
(45) Date of publication and mention of the grant of the patent: **03.05.2017**
(21) Application number: 10747168.2
(22) Date of filing: 12.08.2010
(51) Int. Cl.: A61K 39/395, C07K 16/28, A61K 31/4184, A61P 35/00

(54) **COMBINATION THERAPY OF AN AFUCOSYLATED CD20 ANTIBODY WITH BENDAMUSTINE**
KOMBINATIONSTHERAPIE EINES AFUCOSYLIERTEN CD20-ANTIKÖRPERS UND BENDAMUSTIN
THÉRAPIE COMBINÉE D'UN ANTICORPS CD20 AFUCOSYLATÉ ET DE BENDAMUSTINE

(30) Priority: 14.08.2009 EP 09010489
(43) Date of publication of application: 20.06.2012
(73) Proprietor: Roche Glycart AG, 8952 Schlieren-Zuerich (CH)
(72) Inventor: HERTING, Frank, 82377 Penzberg (DE); KLEIN, Christian, 8906 Bonstetten (CH)
(74) Representative: Burger, Alexander
(86) International application number: PCT/EP2010/004939
(87) International publication number: WO 2011/018224

(56) References cited:
- WO-A1-2010/083365
- WO-A2-2004/103301
- WO-A2-2009/053038
- DATABASE BIOSIS [Online] BIOSCIENCES INFORMATION SERVICE, PHILADELPHIA, PA, US; November 2008 (2008-11), FISCHER KIRSTEN ET AL: "Bendamustine in Combination with Rituximab (BR) for Patients with Relapsed Chronic Lymphocytic Leukemia (CLL): A Multicentre Phase II Trial of the German CLL Study Group (GCLLSG)", XP002559057, Database accession no. PREV200900257032 & BLOOD, vol. 112, no. 11, November 2008 (2008-11), page 128, 50TH ANNUAL MEETING OF THE AMERICAN- SOCIETY-OF-HEMATOLOGY; SAN FRANCISCO, CA, USA; DECEMBER 06 -09, 2008 ISSN: 0006-4971
- UMANA PABLO ET AL.: "GA101, a novel humanized type IICD20 antibody with glycoengineered Fc and enhanced cell death induction, exhibits superior anti-tumour efficacy and superior tissue B-cell depletion in vivo", BLOOD, AMERICAN SOCIETY OF HEMATOLOGY, US, vol. 110, no. 11, abstract:2348, 2007, XP2559271, ISSN: 0006-4971
- UMANA P ET AL: "Novel 3(rd) generation humanized type IICD20 antibody with glycoengineered fc and modified elbow hinge for enhanced ADCC and superior apoptosis induction", BLOOD, AMERICAN SOCIETY OF HEMATOLOGY, US, vol. 108, no. 11, PART 1, 9 December 2006 (2006-12-09), page 72A, XP008087672, ISSN: 0006-4971
- DATABASE BIOSIS [Online] BIOSCIENCES INFORMATION SERVICE, PHILADELPHIA, PA, US; 16 November 2003 (2003-11-16), RUMMEL MATHIAS J ET AL: "Bendamustine and rituximab act synergistically in vitro and are effective in the treatment of relapsed or refractory indolent and mantle-cell lymphomas.", XP002559058, Database accession no. PREV200400172552 & BLOOD, vol. 102, no. 11, 16 November 2003 (2003-11-16), page 104a, 45TH ANNUAL MEETING OF THE AMERICAN SOCIETY OF HEMATOLOGY; SAN DIEGO, CA, USA; DECEMBER 06-09, 2003 ISSN: 0006-4971
- DATABASE BIOSIS [Online] BIOSCIENCES INFORMATION SERVICE, PHILADELPHIA, PA, US; November 2008 (2008-11), SALLES GILLES ANDRE ET AL: "A Phase I/II Study of RO5072759 (GA101) in Patients with Relapsed/Refractory CD20(+) Malignant Disease", XP002559059, Database accession no. PREV200900256936 & BLOOD, vol. 112, no. 11, November 2008 (2008-11), page 93, 50TH ANNUAL MEETING OF THE AMERICAN- SOCIETY-OF-HEMATOLOGY; SAN FRANCISCO, CA, USA; DECEMBER 06 -09, 2008 ISSN: 0006-4971
- ROBAK T: "How to improve the treatment outcome in chronic lymphocytic leukemia?", LEUKEMIA RESEARCH, 13 August 2009 (2009-08-13), pages 1-4, XP002559060, DOI: 10.1016/j.leukres.2009.07.033
- KNAUF W: "Bendamustine in the treatment of chronic lymphocytic leukemia", EXPERT REVIEW OF ANTICANCER THERAPY 2009 EXPERT REVIEWS LTD. GBR, vol. 9, no. 2, 13 August 2009 (2009-08-13) , pages 165-174, XP8115831, ISSN: 1473-7140
- PLOSKER GREG L ET AL: "Bendamustine A Review of its Use in the Management of Indolent Non-Hodgkin Lymphoma", DRUGS, ADIS INTERNATIONAL LTD, NZ, vol. 68, no. 18, 1 January 2008 (2008-01-01), pages 2645-2660, XP009121943, ISSN: 0012-6667
- CHESON BRUCE D ET AL: "Bendamustine: rebirth of an old drug", JOURNAL OF CLINICAL ONCOLOGY, AMERICAN SOCIETY OF CLINICAL ONCOLOGY, US, vol. 27, no. 9, 20 March 2009 (2009-03-20) , pages 1492-1501, XP009124323, ISSN: 0732-183X
- HOFFMANN-LA ROCHE: "A Study of RO5072759 With Chlorambucil in Patients With Previously Untreated Chronic Lymphocytic Leukemia", CLINICAL TRIALS, [Online] 6 November 2009 (2009-11-06), XP002559062, Retrieved from the Internet: URL:http://clinicaltrials.gov/ct2/show/NCT 01010061?intr=ro5072759&rank=4> [retrieved on 2009-12-04]

## Description

The present invention is directed to the combination therapy of an afucosylated CD20 antibody with bendamustine for the treatment of cancer.

### Background of the Invention

### Afucosylated antibodies

Cell-mediated effector functions of monoclonal antibodies can be enhanced by engineering their oligosaccharide component as described in Umaña, P., et al., Nature Biotechnol. 17 (1999) 176-180; and US 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M.R., et al., Glycobiology 5 (1995) 813-822; Jefferis, R., et al., Immunol. Rev. 163 (1998) 59-76; Wright, A., and Morrison, S.L., Trends Biotechnol. 15 (1997) 26-32). Umaña, P., et al., Nature Biotechnol. 17 (1999) 176-180 and WO 99/154342 showed that overexpression in Chinese hamster ovary (CHO) cells of ß(1,4)-N-acetylglucosaminyltransferase III ("GnTIII"), a glycosyltransferase catalyzing the formation of bisected oligosaccharides, significantly increases the in vitro ADCC activity of antibodies. Alterations in the composition of the N297 carbohydrate or its elimination affect also binding to Fc binding to FcyR and C1q (Umaña, P., et al., Nature Biotechnol. 17 (1999) 176-180; Davies, J., et al., Biotechnol. Bioeng. 74 (2001) 288-294; Mimura, Y., et al., J. Biol. Chem. 276 (2001) 45539-45547; Radaev, S., et al., J. Biol. Chem. 276 (2001) 16478-16483; Shields, R.L., et al., J. Biol. Chem. 276 (2001) 6591-6604; Shields, R.L., et al., J. Biol. Chem. 277 (2002) 26733-26740; Simmons, L.C., et al., J. Immunol. Methods 263 (2002) 133-147).

Studies discussing the activities of afucosylated and fucosylated antibodies, including anti-CD20 antibodies, have been reported (e.g., Iida, S., et al., Clin. Cancer Res. 12 (2006) 2879-2887; Natsume, A., et al., J. Immunol. Methods 306 (2005) 93-103; Satoh, M., et al., Expert Opin. Biol. Ther. 6 (2006) 1161-1173 ; Kanda, Y., et al., Biotechnol. Bioeng. 94 (2004) 680-688; Davies, J., et al., Biotechnol. Bioeng. 74 (2001) 288-294.

### CD20 and anti CD20 antibodies

The CD20 molecule (also called human B-lymphocyte-restricted differentiation antigen or Bp35) is a hydrophobic transmembrane protein located on pre-B and mature B lymphocytes that has been described extensively (Valentine, M.A., et al., J. Biol. Chem. 264 (1989) 11282-11287; and Einfeld, D.A., et al., EMBO J. 7 (1988) 711-717; Tedder, T.F., et al., Proc. Natl. Acad. Sci. U.S.A. 85 (1988) 208-12; Stamenkovic, I., et al., J. Exp. Med. 167 (1988) 1975-80; Tedder, T.F., et al., J. Immunol. 142 (1989) 2560-8). CD20 is expressed on greater than 90 % of B cell non-Hodgkin's lymphomas (NHL) (Anderson, K.C., et al., Blood 63 (1984) 1424-1433)) but is not found on hematopoietic stem cells, pro-B cells, normal plasma cells, or other normal tissues (Tedder, T.F., et al., J, Immunol. 135(2) (1985) 973-979).

There exist two different types of anti-CD20 antibodies differing significantly in their mode of CD20 binding and biological activities (Cragg, M.S., et al., Blood 103 (2004) 2738-2743; and Cragg, M.S., et al., Blood 101 (2003) 1045-1051). Type I antibodies, as e.g. rituximab, are potent in complement mediated cytotoxicity, whereas type II antibodies, as e.g. Tositumomab (B1), 11B8, AT80 or humanized B-Ly1 antibodies, effectively initiate target cell death via caspase-independent apoptosis with concomitant phosphatidylserine exposure.

The sharing common features of type I and type II anti-CD20 antibodies are summarized in Table 1.

**Table 1: Properties of type I and type II anti-CD20 antibodies**

| **type I anti-CD20 antibodies** | **type II anti-CD20 antibodies** |
|---|---|
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity (if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

WO2009053038 relates to the combination therapy of a type II anti-CD20 antibody with a proteasome inhibitor.

### Bendamustine

Bendamustine (trade names Ribomustin and Treanda; also known as SDX-105) is a nitrogen mustard used in the treatment of chronic lymphocytic leukemia (CLL) (Kath, R., et al., J. Cancer Res. Clin. Oncol. 127 (2001) 48-54) and non-Hodgkin's lymphoma (NHL). It belongs to the family of drugs called alkylating agents. It is also being studied for the treatment of sarcoma (Bagchi, S., Lancet Oncol. 8 (2007) 674).

Bendamustine has been used as a therapeutic agent with different other agents, including rituximab (Cheson, B.D., et al., J Clin Oncol. 27(9) 2009 1492-501; Knauf, W., Expert Rev Anticancer Ther. (2) 9 (2009) 165-74; Plosker, G.L., et al., Drugs. 68(18) (2008) 2645-60).

### Summary of the Invention

Surprisingly we have now found out that the combination of bendamustine with an afucosylated anti-CD20 antibody, said antibody comprising an amino acid sequence of the variable region of the heavy chain (VH) of SEQ ID NO: 7, and an amino acid sequences of the variable region of the light chain (VL) of SEQ ID NO: 20, showed synergistic (e.g. even more than additive) antiproliferative effects compared to the combination with non-afucosylated CD20 antibody rituximab.

The invention comprises the use of said afucosylated anti-CD20 antibody with an amount of fucose of 60 % or less of the total amount of oligosaccharides (sugars) at Asn297, for the manufacture of a medicament for the treatment of a CD20 expressing cancer in combination with bendamustine.

Another aspect of the invention is said afucosylated anti-CD20 antibody with an amount of fucose of 60 % or less of the total amount of oligosaccharides (sugars) at Asn297, for use in the treatment of a CD20 expressing cancer in combination with bendamustine.

In one embodiment, the amount of fucose is between 40 % and 60 % of the total amount of oligosaccharides (sugars) at Asn297.

In another embodiment, the amount of fucose is 0% of the total amount of oligosaccharides at Asn297.

In one embodiment, the afucosylated anti-CD20 antibody is an IgG1 antibody.

In another embodiment, said CD20 expressing cancer is a B-Cell Non-Hodgkin's lymphoma (NHL).

In one embodiment, the humanized B-Ly1 antibody is administered in a dosage of 800 to 1200 mg on day 1, 8, 15 of a 6-week-dosage-cycle and then in a dosage of 800 to 1200 mg on day 1 of up to five 4-week-dosage-cycles, and bendamustine is administered in a dosage of 80 mg/m2 to 110 mg/ m2 on day 1 and 2 of up to six 4-week-dosage-cycles .

Also disclosed is a composition comprising an anti-CD20 afucosylated antibody with an amount of fucose of 60 % or less, and bendamustine for the treatment of cancer.

### Detailed Description of the Invention

The invention comprises the use of an afucosylated anti-CD20 antibody of IgG1 or IgG3 isotype (preferably of IgG1 isotype)with an amount of fucose of 60 % or less of the total amount of oligosaccharides (sugars) at Asn297, said antibody comprising an amino acid sequence of the variable region of the heavy chain (VH) of SEQ ID NO: 7, and an amino acid sequences of the variable region of the light chain (VL) of SEQ ID NO: 20, for the manufacture of a medicament for the treatment of a CD20 expressing cancer in combination with bendamustine. In one embodiment, the afucosylated anti-CD20 antibody binds CD20 with an KD of 10⁻⁹ M to 10⁻¹³ mol/l.

In one embodiment, the amount of fucose is between 40 % and 60 % of the total amount of oligosaccharides (sugars) at Asn297.

The term "antibody" encompasses the various forms of antibodies including but not being limited to whole antibodies, human antibodies, humanized antibodies and genetically engineered antibodies like monoclonal antibodies, chimeric antibodies or recombinant antibodies as well as fragments of such antibodies as long as the characteristic properties according to the invention are retained. The terms "monoclonal antibody" or "monoclonal antibody composition" as used herein refer to a preparation of antibody molecules of a single amino acid composition. Accordingly, the term "human monoclonal antibody" refers to antibodies displaying a single binding specificity which have variable and constant regions derived from human germline immunoglobulin sequences. In one embodiment, the human monoclonal antibodies are produced by a hybridoma which includes a B cell obtained from a transgenic non-human animal, e.g. a transgenic mouse, having a genome comprising a human heavy chain transgene and a light human chain transgene fused to an immortalized cell.

The term "chimeric antibody" refers to a monoclonal antibody comprising a variable region, i.e., binding region, from one source or species and at least a portion of a constant region derived from a different source or species, usually prepared by recombinant DNA techniques. Chimeric antibodies comprising a murine variable region and a human constant region are especially preferred. Such murine/human chimeric antibodies are the product of expressed immunoglobulin genes comprising DNA segments encoding murine immunoglobulin variable regions and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., Morrison, S.L., et al., Proc. Natl. Acad Sci. USA 81 (1984) 6851-6855; US 5,202,238 and US 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270.

The term "human antibody", as used herein, is intended to include antibodies having variable and constant regions derived from human germline immunoglobulin sequences. Human antibodies are well-known in the state of the art (van Dijk, M.A., and van de Winkel, J.G., Curr. Opin. Pharmacol. 5 (2001) 368-374). Based on such technology, human antibodies against a great variety of targets can be produced. Examples of human antibodies are for example described in Kellermann, S.A., et al., Curr Opin Biotechnol. 13 (2002) 593-597.

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions derived from human germline immunoglobulin sequences in a rearranged form. The recombinant human antibodies according to the invention have been subjected to in vivo somatic hypermutation. Thus, the amino acid sequences of the VH and VL regions of the recombinant antibodies are sequences that, while derived from and related to human germline VH and VL sequences, may not naturally exist within the human antibody germline repertoire in vivo.

As used herein, the term "binding" or "specifically binding" refers to the binding of the antibody to an epitope of the tumor antigen in an in vitro assay, preferably in an plasmon resonance assay (BIAcore, GE-Healthcare Uppsala, Sweden) with purified wild-type antigen. The affinity of the binding is defined by the terms ka (rate constant for the association of the antibody from the antibody/antigen complex), k_{D} (dissociation constant), and K_{D} (k_{D}/ka). Binding or specifically binding means a binding affinity (K_{D}) of 10⁻⁸ mol/l or less, preferably 10⁻⁹ M to 10⁻¹³ mol/l. Thus, an afucosylated antibody according to the invention is specifically binding to the tumor antigen with a binding affinity (K_{D}) of 10⁻⁸ mol/l or less, preferably 10⁻⁹ M to 10⁻¹³ mol/l.

The term "nucleic acid molecule", as used herein, is intended to include DNA molecules and RNA molecules. A nucleic acid molecule may be single-stranded or double-stranded, but preferably is double-stranded DNA.

The "constant domains" are not involved directly in binding the antibody to an antigen but are involved in the effector functions (ADCC, complement binding, and CDC).

The "variable region" (variable region of a light chain (VL), variable region of a heavy chain (VH)) as used herein denotes each of the pair of light and heavy chains which is involved directly in binding the antibody to the antigen. The domains of variable human light and heavy chains have the same general structure and each domain comprises four framework (FR) regions whose sequences are widely conserved, connected by three "hypervariable regions" (or complementarity determining regions, CDRs). The framework regions adopt a b-sheet conformation and the CDRs may form loops connecting the b-sheet structure. The CDRs in each chain are held in their three-dimensional structure by the framework regions and form together with the CDRs from the other chain the antigen binding site.

The terms "hypervariable region" or "antigen-binding portion of an antibody" when used herein refer to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. Especially, CDR3 of the heavy chain is the region which contributes most to antigen binding. CDR and FR regions are determined according to the standard definition of Kabat, et al., Sequences of Proteins of Immunological Interest, 5th Ed. Public Health Service, National Institutes of Health, Bethesda, MD. (1991)) and/or those residues from a "hypervariable loop".

Bendamustine is 4-[5-[Bis(2-chloroethyl)amino]-1-methylbenzimidazol-2-yl]butanoic acid. Trade names are Ribomustin and Treanda; bendamustine is also known as SDX-105). Bendamustine is a nitrogen mustard used in the treatment of chronic lymphocytic leukemia (CLL) (Kath, R., et al., J. Cancer Res. Clin. Oncol. 127 (2001) 48-54) and non-Hodgkin's lymphoma (NHL). It belongs to the family of drugs called alkylating agents. It is also being studied for the treatment of sarcoma (Bagchi, S., Lancet Oncol. 8 (2007) 674).

The term "afucosylated antibody" refers to an antibody of IgG1 or IgG3 isotype (preferably of IgG1 isotype) with an altered pattern of glycosylation in the Fc region at Asn297 having a reduced level of fucose residues. Glycosylation of human IgG1 or IgG3 occurs at Asn297 as core fucosylated bianntennary complex oligosaccharide glycosylation terminated with up to 2 Gal residues. These structures are designated as G0, G1 (α1,6 or α1,3) or G2 glycan residues, depending from the amount of terminal Gal residues (Raju, T.S., BioProcess Int. 1 (2003) 44-53). CHO type glycosylation of antibody Fc parts is e.g. described by Routier, F.H., Glycoconjugate J. 14 (1997) 201-207. Antibodies which are recombinantly expressed in non glycomodified CHO host cells usually are fucosylated at Asn297 in an amount of at least 85 %. It should be understood that the term an afucosylated antibody as used herein includes an antibody having no fucose in its glycosylation pattern. It is commonly known that typical glycosylated residue position in an antibody is the asparagine at position 297 according to the EU numbering system ("Asn297").

The "EU numbering system" or "EU index" is generally used when referring to a residue in an immunoglobulin heavy chain constant region (e.g., the EU index reported in Kabat et al., Sequences of Proteins of Immunological Interest, 5th Ed. Public Health Service, National Institutes of Health, Bethesda, MD (1991) expressly incorporated herein by reference).

Thus an afucosylated antibody according to the invention means an antibody of IgG1 or IgG3 isotype(preferably of IgG1 isotype) wherein the amount of fucose is 60 % or less of the total amount of oligosaccharides (sugars) at Asn297 (which means that at least 40 % or more of the oligosaccharides of the Fc region at Asn297 are afucosylated). In one embodiment the amount of fucose is between 40 % and 60 % of the oligosaccharides of the Fc region at Asn297. In another embodiment the amount of fucose is 50 % or less, and in still another embodiment the amount of fucose is 30 % or less of the oligosaccharides of the Fc region at Asn297. In an alternative embodiment, the amount of fucose is 0% of the oligosaccharides of the Fc region at Asn297. According to the invention "amount of fucose" means the amount of said oligosaccharide (fucose) within the oligosaccharide (sugar) chain at Asn297, related to the sum of all oligosaccharides (sugars) attached to Asn 297 (e. g. complex, hybrid and high mannose structures) measured by MALDI-TOF mass spectrometry and calculated as average value (a detailed procedure to determine the amount of fucose, is described e.g. in WO 2008/077546). Furthermore, in one embodiment, the oligosaccharides of the Fc region are bisected. The afucosylated antibody according to the invention can be expressed in a glycomodified host cell engineered to express at least one nucleic acid encoding a polypeptide having GnTIII activity in an amount sufficient to partially fucosylate the oligosaccharides in the Fc region. In one embodiment, the polypeptide having GnTIII activity is a fusion polypeptide. Alternatively α1,6-fucosyltransferase activity of the host cell can be decreased or eliminated according to US 6,946,292 to generate glycomodified host cells. The amount of antibody fucosylation can be predetermined e.g. either by fermentation conditions (e.g. fermentation time) or by combination of at least two antibodies with different fucosylation amount. Such afucosylated antibodies and respective glycoengineering methods are described in WO 2005/044859, WO 2004/065540, WO 2007/031875, Umana, P., et al., Nature Biotechnol. 17 (1999) 176-180, WO 99/154342, WO 2005/018572, WO 2006/116260, WO 2006/114700, WO 2005/011735, WO 2005/027966, WO 97/028267, US2006/0134709, US2005/0054048, US2005/0152894, WO 2003/035835, WO 2000/061739. These glycoengineered antibodies have an increased ADCC. Other glycoengineering methods yielding afucosylated antibodies according to the invention are described e.g. in Niwa, R., et al., J. Immunol. Methods 306 (2005) 151-160; Shinkawa, T., et al., J Biol Chem, 278 (2003) 3466-3473; WO 03/055993 or US2005/0249722.

Thus one aspect of the invention is the use of an afucosylated anti-CD20 antibody of IgG1 or IgG3 isotype (preferably of IgG1 isotype) specifically binding to CD20 with an amount of fucose of 60 % or less of the total amount of oligosaccharides (sugars) at Asn297, said antibody comprising an amino acid sequence of the variable region of the heavy chain (VH) of SEQ ID NO: 7, and an amino acid sequences of the variable region of the light chain (VL) of SEQ ID NO: 20, for the manufacture of a medicament for the treatment of cancer in combination with bendamustine. In one embodiment, the amount of fucose is between 40 % and 60 % of the total amount of oligosaccharides (sugars) at Asn297.

CD20 (also known as B-lymphocyte antigen CD20, B-lymphocyte surface antigen B1, Leu-16, Bp35, BM5, and LF5; the sequence is characterized by the SwissProt database entry P11836) is a hydrophobic transmembrane protein with a molecular weight of approximately 35 kD located on pre-B and mature B lymphocytes. (Valentine, M.A., et al., J. Biol. Chem. 264(19) (1989) 11282-11287; Tedder, T.F., et al., Proc. Natl. Acad. Sci. U.S.A. 85 (1988) 208-212; Stamenkovic, I., et al., J. Exp. Med. 167 (1988) 1975-80; Einfeld, D.A., et al., EMBO J. 7 (1988) 711-7; Tedder, T.F., et al., J. Immunol. 142 (1989) 2560-2568). The corresponding human gene is Membrane-spanning 4-domains, subfamily A, member 1, also known as MS4A1. This gene encodes a member of the membrane-spanning 4A gene family. Members of this nascent protein family are characterized by common structural features and similar intron/exon splice boundaries and display unique expression patterns among hematopoietic cells and nonlymphoid tissues. This gene encodes the B-lymphocyte surface molecule which plays a role in the development and differentiation of B-cells into plasma cells. This family member is localized to 11q12, among a cluster of family members. Alternative splicing of this gene results in two transcript variants which encode the same protein.

The terms "CD20" and "CD20 antigen" are used interchangeably herein, and include any variants, isoforms and species homologs of human CD20 which are naturally expressed by cells or are expressed on cells transfected with the CD20 gene. Binding of an antibody of the invention to the CD20 antigen mediate the killing of cells expressing CD20 (e.g., a tumor cell) by inactivating CD20. The killing of the cells expressing CD20 may occur by one or more of the following mechanisms: Cell death/apoptosis induction, ADCC and CDC.

Synonyms of CD20, as recognized in the art, include B-lymphocyte antigen CD20, B-lymphocyte surface antigen B1, Leu-16, Bp35, BM5, and LF5.

The term "anti-CD20 antibody" according to the invention is an antibody that binds specifically to CD20 antigen. Depending on binding properties and biological activities of anti-CD20 antibodies to the CD20 antigen, two types of anti-CD20 antibodies (type I and type II anti-CD20 antibodies) can be distinguished according to Cragg, M.S., et al., Blood 103 (2004) 2738-2743; and Cragg, M.S., et al., Blood 101 (2003) 1045-1051, see Table 2.

**Table 2: Properties of type I and type II anti-CD20 antibodies**

| **type I anti-CD20 antibodies** | **type II anti-CD20 antibodies** |
|---|---|
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity (if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

Examples of type II anti-CD20 antibodies include e.g. humanized B-Ly1 antibody IgG1 (a chimeric humanized IgG1 antibody as disclosed in WO 2005/044859), 11B8 IgG1 (as disclosed in WO 2004/035607), and AT80 IgG1. Typically type II anti-CD20 antibodies of the IgG1 isotype show characteristic CDC properties. Type II anti-CD20 antibodies have a decreased CDC (if IgG1 isotype) compared to type I antibodies of the IgG1 isotype.

Examples of type I anti-CD20 antibodies include e.g. rituximab, HI47 IgG3 (ECACC, hybridoma), 2C6 IgG1 (as disclosed in WO 2005/103081), 2F2 IgG1 (as disclosed and WO2004/035607 and WO2005/103081) and 2H7 IgG1 (as disclosed in WO 2004/056312).

The afucosylated anti-CD20 antibodies according to the invention is in one embodiment a type II anti-CD20 antibody, in a more specific embodiment, the type II anti-CD20 antibody is an afucosylated humanized B-Ly1 antibody.

The afucosylated anti-CD20 antibodies according to the invention have an increased antibody dependent cellular cytotoxicity (ADCC) unlike anti-CD20 antibodies having no reduced fucose.

By "afucosylated anti-CD20 antibody with increased antibody dependent cellular cytotoxicity (ADCC)" is meant an afucosylated anti-CD20 antibody, as that term is defined herein, having increased ADCC as determined by any suitable method known to those of ordinary skill in the art. One accepted in vitro ADCC assay is as follows:
1) the assay uses target cells that are known to express the target antigen recognized by the antigen-binding region of the antibody;
2) the assay uses human peripheral blood mononuclear cells (PBMCs), isolated from blood of a randomly chosen healthy donor, as effector cells;
3) the assay is carried out according to following protocol:
   i) the PBMCs are isolated using standard density centrifugation procedures and are suspended at 5 x 10⁶ cells/ml in RPMI cell culture medium;
   ii) the target cells are grown by standard tissue culture methods, harvested from the exponential growth phase with a viability higher than 90 %, washed in RPMI cell culture medium, labeled with 100 micro-Curies of ⁵¹Cr, washed twice with cell culture medium, and resuspended in cell culture medium at a density of 10⁵ cells/ml;
   iii) 100 microliters of the final target cell suspension above are transferred to each well of a 96-well microtiter plate;
   iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;
   v) for the maximum release (MR) controls, 3 additional wells in the plate containing the labeled target cells, receive 50 microliters of a 2 % (VN) aqueous solution of non-ionic detergent (Nonidet, Sigma, St. Louis), instead of the antibody solution (point iv above);
   vi) for the spontaneous release (SR) controls, 3 additional wells in the plate containing the labeled target cells, receive 50 microliters of RPMI cell culture medium instead of the antibody solution (point iv above);
   vii) the 96-well microtiter plate is then centrifuged at 50 x g for 1 minute and incubated for 1 hour at 4 °C;
   viii) 50 microliters of the PBMC suspension (point i above) are added to each well to yield an effector:target cell ratio of 25: 1 and the plates are placed in an incubator under 5 % CO2 atmosphere at 37 °C for 4 hours;
   ix) the cell-free supernatant from each well is harvested and the experimentally released radioactivity (ER) is quantified using a gamma counter;
   x) the percentage of specific lysis is calculated for each antibody concentration according to the formula (ER-MR)/(MR-SR) x 100, where ER is the average radioactivity quantified (see point ix above) for that antibody concentration, MR is the average radioactivity quantified (see point ix above) for the MR controls (see point V above), and SR is the average radioactivity quantified (see point ix above) for the SR controls (see point vi above);
4) "increased ADCC" is defined as either an increase in the maximum percentage of specific lysis observed within the antibody concentration range tested above, and/or a reduction in the concentration of antibody required to achieve one half of the maximum percentage of specific lysis observed within the antibody concentration range tested above. The increase in ADCC is relative to the ADCC, measured with the above assay, mediated by the same antibody, produced by the same type of host cells, using the same standard production, purification, formulation and storage methods, which are known to those skilled in the art, but that has not been produced by host cells engineered to overexpress GnTIII.

Said "increased ADCC" can be obtained by glycoengineering of said antibodies, that means enhance said natural, cell-mediated effector functions of monoclonal antibodies by engineering their oligosaccharide component as described in Umana, P., et al., Nature Biotechnol. 17 (1999) 176-180 and US 6,602,684.

The term "complement-dependent cytotoxicity (CDC)" refers to lysis of human tumor target cells by the antibody according to the invention in the presence of complement. CDC is measured preferably by the treatment of a preparation of CD20 expressing cells with an anti-CD20 antibody according to the invention in the presence of complement. CDC is found if the antibody induces at a concentration of 100 nM the lysis (cell death) of 20 % or more of the tumor cells after 4 hours. The assay is performed preferably with ⁵¹Cr or Eu labeled tumor cells and measurement of released ⁵¹Cr or Eu. Controls include the incubation of the tumor target cells with complement but without the antibody.

The "rituximab" antibody (reference antibody; example of a type I anti-CD20 antibody) is a genetically engineered chimeric human gamma 1 murine constant domain containing monoclonal antibody directed against the human CD20 antigen. This chimeric antibody contains human gamma 1 constant domains and is identified by the name "C2B8" in US 5,736,137 (Andersen et. al.) issued on April 17, 1998, assigned to IDEC Pharmaceuticals Corporation. Rituximab is approved for the treatment of patients with relapsed or refracting low-grade or follicular, CD20 positive, B cell non-Hodgkin's lymphoma. In vitro mechanism of action studies have shown that rituximab exhibits human complement--dependent cytotoxicity (CDC) (Reff, M.E., et. al., Blood 83(2) (1994) 435-445). Additionally, it exhibits significant activity in assays that measure antibody-dependent cellular cytotoxicity (ADCC). Rituximab is not afucosylated.

| Antibody | Amount of fucose |
|---|---|
| Rituximab (non-afucosylated) | >85 % |
| Wild type afucosylated glyco-engineered humanized B-Ly1 (B-HH6-B-KV1) (non-afucosylated) | >85 % |
| afucosylated glyco-engineered humanized B-Ly1 (B-HH6-B-KV1 GE) | 45-50 % |

The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (variable region of the murine heavy chain (VH): SEQ ID NO: 1; variable region of the murine light chain (VL): SEQ ID NO: 2- see Poppema, S. and Visser, L., Biotest Bulletin 3 (1987) 131-139) by chimerization with a human constant domain from IgG1 and following humanization (see WO 2005/044859 and WO 2007/031875). These "humanized B-Ly1 antibodies" are disclosed in detail in WO 2005/044859 and WO 2007/031875.

The "humanized B-Ly1 antibody" disclosed herein has variable region of the heavy chain (VH) selected from group of SEQ ID NO:3 to SEQ ID NO:20 (B-HH2 to B-HH9 and B-HL8 to B-HL17 of WO 2005/044859 and WO 2007/031875). Especially preferred are Seq. ID No. 3, 4, 7, 9, 11, 13 and 15 (B-HH2, B-HH3, B-HH6, B-HH8, B-HL8, B-HL11 and B-HL13 of WO2005/044859 and WO 2007/031875). In one specific embodiment, the "humanized B-Ly1 antibody" has variable region of the light chain (VL) of SEQ ID No. 20 (B-KV1 of WO 2005/044859 and WO 2007/031875). In a specific embodiment, the "humanized B-Ly1 antibody" has a variable region of the heavy chain (VH) of SEQ ID NO:7 (B-HH6 of WO 2005/044859 and WO 2007/031875) and a variable region of the light chain (VL) of SEQ ID No. 20 (B-KV1 of WO 2005/044859 and WO 2007/031875). Furthermore, in one embodiment, the humanized B-Ly1 antibody is an IgG1 antibody. According to the invention such afucosylated humanized B-Ly1 antibodies are glycoengineered (GE) in the Fc region according to the procedures described in WO 2005/044859, WO 2004/065540, WO 2007/031875, Umana, P., et al., Nature Biotechnol. 17 (1999) 176-180 and WO 99/154342. In one embodiment of the invention, the anti-CD20 antibody used is afucosylated glyco-engineered humanized B-Ly1 known as B-HH6-B-KV1 GE. Such glycoengineered humanized B-Ly1 antibodies have an altered pattern of glycosylation in the Fc region, preferably having a reduced level of fucose residues. In one embodiment, the amount of fucose is 60 % or less of the total amount of oligosaccharides at Asn297 (in one embodiment the amount of fucose is between 40 % and 60 %, in another embodiment the amount of fucose is 50 % or less, and in still another embodiment the amount of fucose is 30 % or less, and in yet another embodiment, the amount of fucose is 0%). Furthermore, in one specific embodiment, the oligosaccharides of the Fc region are bisected. These glycoengineered humanized B-Ly1 antibodies have an increased ADCC.

The oligosaccharide component can significantly affect properties relevant to the efficacy of a therapeutic glycoprotein, including physical stability, resistance to protease attack, interactions with the immune system, pharmacokinetics, and specific biological activity. Such properties may depend not only on the presence or absence, but also on the specific structures, of oligosaccharides. Some generalizations between oligosaccharide structure and glycoprotein function can be made. For example, certain oligosaccharide structures mediate rapid clearance of the glycoprotein from the bloodstream through interactions with specific carbohydrate binding proteins, while others can be bound by antibodies and trigger undesired immune reactions. (Jenkins, N., et al., Nature Biotechnol. 14 (1996) 975-981).

Mammalian cells are the excellent hosts for production of therapeutic glycoproteins, due to their capability to glycosylate proteins in the most compatible form for human application. (Cumming, D.A., et al., Glycobiology 1 (1991) 115-30; Jenkins, N., et al., Nature Biotechnol. 14 (1996) 975-981). Bacteria very rarely glycosylate proteins, and like other types of common hosts, such as yeasts, filamentous fungi, insect and plant cells, yield glycosylation patterns associated with rapid clearance from the blood stream, undesirable immune interactions, and in some specific cases, reduced biological activity. Among mammalian cells, Chinese hamster ovary (CHO) cells have been most commonly used during the last two decades. In addition to giving suitable glycosylation patterns, these cells allow consistent generation of genetically stable, highly productive clonal cell lines. They can be cultured to high densities in simple bioreactors using serum free media, and permit the development of safe and reproducible bioprocesses. Other commonly used animal cells include baby hamster kidney (BHK) cells, NSO- and SP2/0-mouse myeloma cells. More recently, production from transgenic animals has also been tested. (Jenkins, N., et al., Nature Biotechnol. 14 (1996) 975-981).

All antibodies contain carbohydrate structures at conserved positions in the heavy chain constant regions, with each isotype possessing a distinct array of N-linked carbohydrate structures, which variably affect protein assembly, secretion or functional activity. (Wright, A., and Monison, S.L., Trends Biotech. 15 (1997) 26-32). The structure of the attached N-linked carbohydrate varies considerably, depending on the degree of processing, and can include high-mannose, multiply-branched as well as biantennary complex oligosaccharides. (Wright, A., and Morrison, S.L., Trends Biotech. 15 (1997) 26-32). Typically, there is heterogeneous processing of the core oligosaccharide structures attached at a particular glycosylation site such that even monoclonal antibodies exist as multiple glycoforms. Likewise, it has been shown that major differences in antibody glycosylation occur between cell lines, and even minor differences are seen for a given cell line grown under different culture conditions. (Lifely, M.R., et al., Glycobiology 5(8) (1995) 813-22

One way to obtain large increases in potency, while maintaining a simple production process and potentially avoiding significant, undesirable side effects, is to enhance the natural, cell-mediated effector functions of monoclonal antibodies by engineering their oligosaccharide component as described in Umana, P., et al., Nature Biotechnol. 17 (1999) 176-180 and US 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M.R., et al., Glycobiology 5 (1995) 813-822; Jefferis, R., et al., Immunol. Rev. 163 (1998) 59-76; Wright, A., and Morrison, S.L., Trends Biotechnol. 15 (1997) 26-32).

It was previously shown that overexpression in Chinese hamster ovary (CHO) cells of ß(1,4)-N-acetylglucosaminyltransferase I11 ("GnTII17y"), a glycosyltransferase catalyzing the formation of bisected oligosaccharides, significantly increases the in vitro ADCC activity of an antineuroblastoma chimeric monoclonal antibody (chCE7) produced by the engineered CHO cells. (See Umana, P., et al., Nature Biotechnol. 17 (1999) 176-180; and WO 99/154342, the entire contents of which are hereby incorporated by reference). The antibody chCE7 belongs to a large class of unconjugated monoclonal antibodies which have high tumor affinity and specificity, but have too little potency to be clinically useful when produced in standard industrial cell lines lacking the GnTIII enzyme (Umana, P., et al., Nature Biotechnol. 17 (1999) 176-180). That study was the first to show that large increases of ADCC activity could be obtained by engineering the antibody producing cells to express GnTIII, which also led to an increase in the proportion of constant region (Fc)-associated, bisected oligosaccharides, including bisected, non-fucosylated oligosaccharides, above the levels found in naturally-occurring antibodies.

The term "cancer" as used herein includes lymphomas, lymphocytic leukemias, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, including refractory versions of any of the above cancers, or a combination of one or more of the above cancers. In one embodiment, the term cancer refers to a CD20 expressing cancer.

The term "expression of the CD20" antigen is intended to indicate an significant level of expression of the CD20 antigen in a cell, preferably on the cell surface of a T- or B- cell, more preferably a B-cell, from a tumor or cancer, respectively, preferably a non-solid tumor. Patients having a "CD20 expressing cancer" can be determined by standard assays known in the art. For example, CD20 antigen expression can be measured using immunohistochemical (IHC) detection, FACS or via PCR-based detection of the corresponding mRNA.

The term "CD20 expressing cancer" as used herein refers to all cancers in which the cancer cells show an expression of the CD20 antigen. Preferably CD20 expressing cancer as used herein refers to lymphomas (preferably B-cell Non-Hodgkin's lymphomas (NHL)) and lymphocytic leukemias. Such lymphomas and lymphocytic leukemias include e.g. a) follicular lymphomas, b) Small Non-Cleaved Cell Lymphomas/ Burkitt's lymphoma (including endemic Burkitt's lymphoma, sporadic Burkitt's lymphoma and Non-Burkitt's lymphoma) c) marginal zone lymphomas (including extranodal marginal zone B cell lymphoma (Mucosa-associated lymphatic tissue lymphomas, MALT), nodal marginal zone B cell lymphoma and splenic marginal zone lymphoma), d) Mantle cell lymphoma (MCL), e) Large Cell Lymphoma (including B-cell diffuse large cell lymphoma (DLCL), Diffuse Mixed Cell Lymphoma, Immunoblastic Lymphoma, Primary Mediastinal B-Cell Lymphoma, Angiocentric Lymphoma-Pulmonary B-Cell Lymphoma) f) hairy cell leukemia, g) lymphocytic lymphoma, waldenstrom's macroglobulinemia, h) acute lymphocytic leukemia (ALL), chronic lymphocytic leukemia (CLL)/ small lymphocytic lymphoma (SLL), B-cell prolymphocytic leukemia, i) plasma cell neoplasms, plasma cell myeloma, multiple myeloma, plasmacytoma j) Hodgkin's disease.

In one further embodiment, the CD20 expressing cancer is a B-cell Non-Hodgkin's lymphomas (NHL). In another embodiment, the CD20 expressing cancer is a Mantle cell lymphoma (MCL), acute lymphocytic leukemia (ALL), chronic lymphocytic leukemia (CLL), B-cell diffuse large cell lymphoma (DLCL), Burkitt's lymphoma, hairy cell leukemia, follicular lymphoma, multiple myeloma, marginal zone lymphoma, post transplant lymphoproliferative disorder (PTLD),

HIV associated lymphoma, waldenstrom's macroglobulinemia, or primary CNS lymphoma.

The term "a method of treating" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action.

The terms "co-administration" or "co-administering " refer to the administration of said afucosylated anti-CD20, and bendamustine as one single formulation or as two separate formulations. The co-administration can be simultaneous or sequential in either order, wherein preferably there is a time period while both (or all) active agents simultaneously exert their biological activities. Said anti-CD20 afucosylated antibody and bendamustine are co-administered either simultaneously or sequentially (e.g. via an intravenous (i.v.) through a continuous infusion (one for the anti-CD20 antibody and eventually one for bendamustine). When both therapeutic agents are co-administered sequentially the dose is administered either on the same day in two separate administrations, or one of the agents is administered on day 1 and the second is co-administered on day 2 to day 7, preferably on day 2 to 4. Thus the term "sequentially" means within 7 days after the dose of the first component (bendamustine or antibody), preferably within 4 days after the dose of the first component; and the term "simultaneously" means at the same time. The terms "co-administration" with respect to the maintenance doses of said afucosylated anti-CD20 antibody and bendamustine mean that the maintenance doses can be either co-administered simultaneously, if the treatment cycle is appropriate for both drugs, e.g. every week. Or bendamustine is e.g. administered e.g. every first to third day and said afucosylated antibody is administered every week. Or the maintenance doses are co-administered sequentially, either within one or within several days.

It is self-evident that the antibodies are administered to the patient in a "therapeutically effective amount" (or simply "effective amount") which is the amount of the respective compound or combination that will elicit the biological or medical response of a tissue, system, animal or human that is being sought by the researcher, veterinarian, medical doctor or other clinician.

The amount of co-administration of said anti-CD20 afucosylated antibody and bendamustine and the timing of co-administration will depend on the type (species, gender, age, weight, etc.) and condition of the patient being treated and the severity of the disease or condition being treated. Said afucosylated anti-CD20 antibody and bendamustine are suitably co-administered to the patient at one time or over a series of treatments.

If the administration is intravenous the initial infusion time for said afucosylated anti-CD20 antibody or bendamustine may be longer than subsequent infusion times, for instance approximately 90 minutes for the initial infusion, and approximately 30 minutes for subsequent infusions (if the initial infusion is well tolerated).

Depending on the type and severity of the disease, about 1 µg /kg to 50 mg/kg (e.g. 0.1-20 mg/kg) of said afucosylated anti-CD20 antibody and 1 µg /kg to 50 mg/kg (e.g. 0.1-20 mg/kg) of bendamustine is an initial candidate dosage for co-administration of both drugs to the patient. In one embodiment the preferred dosage of said afucosylated anti-CD20 antibody (preferably the afucosylated humanized B-Ly1 antibody) will be in the range from about 0.05mg/kg to about 30mg/kg. Thus, one or more doses of about 0.5mg/kg, 2.0mg/kg, 4.0mg/kg, 10mg/kg or 30mg/kg (or any combination thereof) may be co-administered to the patient. In one embodiment, the dosage of bendamustine will be in the range from 0.01 mg/kg to about 30 mg/kg, e.g. 0.1 mg/kg to 10.0mg/kg. Depending on the type (species, gender, age, weight, etc.) and condition of the patient and on the type of afucosylated anti-CD20 antibody , the dosage and the administration schedule of said afucosylated antibody and bedamustine can differ, e.g., the said afucosylated anti-CD20 antibody may be administered e.g. every one to three weeks and bendamustine may be administered daily or every 2 to 10 days. An initial higher loading dose, followed by one or more lower doses may also be administered.

In one preferred embodiment the preferred dosage of said afucosylated anti-CD20 antibody (preferably the afucosylated humanized B-Ly1 antibody) will be 800 to 1200 mg on day 1, 8, 15 of a 6-week-dosage-cycle and then in a dosage of 800 to 1200 mg on day 1 of up to five 4-week-dosage-cycles and the preferred dosage of bendamustine will be, e.g, 80 mg/m2 to 110 mg/ m2 (in one embodiment 110 mg/ m2, in another embodiment 90 mg/m2) on day 1 and 2 (infused intravenously over 30 minutes on days 1 and 2) of up to six 4-week-dosage-cycles. Alternatively the dosage of said afucosylated anti-CD20 antibody can be 800 to 1200 mg (e.g., 1000 mg) on day 1 up to eight 3-week-dosage-cycles.

In one embodiment, the medicament is useful for preventing or reducing metastasis or further dissemination in such a patient suffering from cancer, preferably CD20 expressing cancer. The medicament is useful for increasing the duration of survival of such a patient, increasing the progression free survival of such a patient, increasing the duration of response, resulting in a statistically significant and clinically meaningful improvement of the treated patient as measured by the duration of survival, progression free survival, response rate or duration of response. In a specific embodiment, the medicament is useful for increasing the response rate in a group of patients.

In the context of this invention, additional other cytotoxic, chemotherapeutic or anti-cancer agents, or compounds that enhance the effects of such agents (e.g. cytokines) may be used in the afucosylated anti-CD20 antibody and bendamustine combination treatment of cancer. Such molecules are suitably present in combination in amounts that are effective for the purpose intended. In one embodiment, the said afucosylated anti-CD20 antibody and bendamustine combination treatment is used without such additional cytotoxic, chemotherapeutic or anti-cancer agents, or compounds that enhance the effects of such agents.

Such agents include, for example: alkylating agents or agents with an alkylating action, such as cyclophosphamide (CTX; e.g. cytoxan®), chlorambucil (CHL; e.g. leukeran®), cisplatin (CisP; e.g. platinol®) busulfan (e.g. myleran®), melphalan, carmustine (BCNU), streptozotocin, triethylenemelamine (TEM), mitomycin C, and the like; anti-metabolites, such as methotrexate (MTX), etoposide (VP16; e.g. vepesid®), 6-mercaptopurine (6MP), 6-thiocguanine (6TG), cytarabine (Ara-C), 5-fluorouracil (5-FU), capecitabine (e.g. Xeloda®), dacarbazine (DTIC), and the like; antibiotics, such as actinomycin D, doxorubicin (DXR; e.g. adriamycin®), daunorubicin (daunomycin), bleomycin, mithramycin and the like; alkaloids, such as vinca alkaloids such as vincristine (VCR), vinblastine, and the like; and other antitumor agents, such as paclitaxel (e.g. taxol®) and paclitaxel derivatives, the cytostatic agents, glucocorticoids such as dexamethasone (DEX; e.g. decadron®) and corticosteroids such as prednisone, nucleoside enzyme inhibitors such as hydroxyurea, amino acid depleting enzymes such as asparaginase, leucovorin and other folic acid derivatives, and similar, diverse antitumor agents. The following agents may also be used as additional agents: amifostine (e.g. ethyol®), dactinomycin, mechlorethamine (nitrogen mustard), streptozocin, cyclophosphamide, lomustine (CCNU), doxorubicin lipo (e.g. doxil®), gemcitabine (e.g. gemzar®), daunorubicin lipo (e.g. daunoxome®), procarbazine, mitomycin, docetaxel (e.g. taxotere®), aldesleukin, carboplatin, oxaliplatin, cladribine, camptothecin, CPT 11 (irinotecan), 10-hydroxy 7-ethyl-camptothecin (SN38), floxuridine, fludarabine, ifosfamide, idarubicin, mesna, interferon beta, interferon alpha, mitoxantrone, topotecan, leuprolide, megestrol, melphalan, mercaptopurine, plicamycin, mitotane, pegaspargase, pentostatin, pipobroman, plicamycin, tamoxifen, teniposide, testolactone, thioguanine, thiotepa, uracil mustard, vinorelbine, chlorambucil. Preferably the afucosylated anti-CD20 antibody and bendamustine combination treatment is used without such additional agents.

The use of the cytotoxic and anticancer agents described above as well as antiproliferative target-specific anticancer drugs like protein kinase inhibitors in chemotherapeutic regimens is generally well characterized in the cancer therapy arts, and their use herein falls under the same considerations for monitoring tolerance and effectiveness and for controlling administration routes and dosages, with some adjustments. For example, the actual dosages of the cytotoxic agents may vary depending upon the patient's cultured cell response determined by using histoculture methods. Generally, the dosage will be reduced compared to the amount used in the absence of additional other agents.

Typical dosages of an effective cytotoxic agent can be in the ranges recommended by the manufacturer, and where indicated by in vitro responses or responses in animal models, can be reduced by up to about one order of magnitude concentration or amount. Thus, the actual dosage will depend upon the judgment of the physician, the condition of the patient, and the effectiveness of the therapeutic method based on the in vitro responsiveness of the primary cultured malignant cells or histocultured tissue sample, or the responses observed in the appropriate animal models.

In the context of this invention, an effective amount of ionizing radiation may be carried out and/or a radiopharmaceutical may be used in addition to the afucosylated anti-CD20 antibody and bendamustine combination treatment of CD20 expressing cancer. The source of radiation can be either external or internal to the patient being treated. When the source is external to the patient, the therapy is known as external beam radiation therapy (EBRT). When the source of radiation is internal to the patient, the treatment is called brachytherapy (BT). Radioactive atoms for use in the context of this invention can be selected from the group including, but not limited to, radium, cesium-137, iridium-192, americium-241, gold-198, cobalt-57, copper-67, technetium-99, iodine-123, iodine-131, and indium-111. Is also possible to label the antibody with such radioactive isotopes. Preferably the afucosylated anti-CD20 antibody and bendamustine combination treatment is used without such ionizing radiation.

Radiation therapy is a standard treatment for controlling unresectable or inoperable tumors and/or tumor metastases. Improved results have been seen when radiation therapy has been combined with chemotherapy. Radiation therapy is based on the principle that high-dose radiation delivered to a target area will result in the death of reproductive cells in both tumor and normal tissues. The radiation dosage regimen is generally defined in terms of radiation absorbed dose (Gy), time and fractionation, and must be carefully defined by the oncologist. The amount of radiation a patient receives will depend on various considerations, but the two most important are the location of the tumor in relation to other critical structures or organs of the body, and the extent to which the tumor has spread. A typical course of treatment for a patient undergoing radiation therapy will be a treatment schedule over a 1 to 6 week period, with a total dose of between 10 and 80 Gy administered to the patient in a single daily fraction of about 1.8 to 2.0 Gy, 5 days a week. In a preferred embodiment of this invention there is synergy when tumors in human patients are treated with the combination treatment of the invention and radiation. In other words, the inhibition of tumor growth by means of the agents comprising the combination of the invention is enhanced when combined with radiation, optionally with additional chemotherapeutic or anticancer agents. Parameters of adjuvant radiation therapies are, for example, contained in WO 99/60023.

The afucosylated anti-CD20 antibodies can be administered to a patient according to known methods, such as by intravenous administration as a bolus or by continuous infusion over a period of time, by intramuscular, intraperitoneal, intracerobrospinal, subcutaneous, intra-articular, intrasynovial, or intrathecal routes. In one embodiment, such antibodies are administered by intravenous or subcutaneous administration.

Bendamustine can be administered to a patient according to known methods, e.g. by intravenous administration as a bolus or by continuous infusion over a period of time, by intramuscular, intraperitoneal, intracerobrospinal, subcutaneous, intra-articular, intrasynovial, intrathecal, or peroral routes. According to one embodiment, such antibody is administered by intravenous or intraperitoneal administration.

As used herein, a "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention is contemplated. Supplementary active compounds can also be incorporated into the compositions.

### Pharmaceutical Compositions

Pharmaceutical compositions can be obtained by processing the anti-CD20 antibody and/or bendamustine according to this invention with pharmaceutically acceptable, inorganic or organic carriers. Lactose, corn starch or derivatives thereof, talc, stearic acids or it's salts and the like can be used, for example, as such carriers for tablets, coated tablets, dragees and hard gelatine capsules. Suitable carriers for soft gelatine capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Disclosed is a composition comprising both said afucosylated anti-CD20 antibody with an amount of fucose is 60 % or less (in one embodiment, said antibody is afucosylated humanized B-Ly1 antibody) and bendamustine for use in the treatment of cancer, in particular, CD20 expressing cancer.

Said pharmaceutical composition may further comprise one or more pharmaceutically acceptable carriers.

The present invention further discloses a pharmaceutical composition, in particular for use in cancer, comprising (i) an effective first amount of an afucosylated anti-CD20 antibody with an amount of fucose is 60 % or less (in one embodiment, an afucosylated humanized B-Ly1 antibody), and (ii) an effective second amount of bendamustine. Such composition optionally comprises pharmaceutically acceptable carriers and / or excipients.

Pharmaceutical compositions of the afucosylated anti-CD20 antibody alone are prepared for storage by mixing an antibody having the desired degree of purity with optional pharmaceutically acceptable carriers, excipients or stabilizers (Remington's Pharmaceutical Sciences 16th edition, Osol, A. (ed.) (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

Pharmaceutical compositions of bendamustine can be similar to those describe above for the afucosylated anti-CD20 antibody.

In one further embodiment of the invention, afucosylated anti-CD20 antibody and bendamustine are formulated in two separate formulations..

The active ingredients may also be entrapped in microcapsules prepared, for example, by coacervation techniques or by interracial polymerization, for example, hydroxymethylcellulose or gelatin-microcapsules and poly- (methylmethacylate) microcapsules, respectively, in colloidal drug delivery systems (for example, liposomes, albumin microspheres, microemulsions, nano- particles and nanocapsules) or in macroemulsions. Such techniques are disclosed in Remington's Pharmaceutical Sciences 16th edition, Osol, A. (ed.) (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (US 3,773,919), copolymers of L-glutamic acid and gamma-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes.

Also disclosed a method for the treatment of cancer, comprising administering to a patient in need of such treatment (i) an effective first amount of an afucosylated anti-CD20 antibody with an amount of fucose is 60 % or less, (in one embodiment, an afucosylated humanized B-Ly1 antibody); and (ii) an effective second amount of bendamustine.

In one embodiment, the amount of fucose of is between 40 % and 60 %.

Preferably said cancer is a CD20 expressing cancer.

Preferably said CD20 expressing cancer is a B-Cell Non-Hodgkin's lymphoma (NHL).

Preferably said afucosylated anti-CD20 antibody is a type II anti-CD20 antibody.

Preferably said antibody is a humanized B-Ly1 antibody.

Preferably said humanized B-Ly1 antibody is administered in a dosage of 800 to 1200 mg on day 1, 8, 15 of a 6-week-dosage-cycle and then in a dosage of 800 to 1200 mg on day 1 of up to five 4-week-dosage-cycles, and bendamustine is administered in a dosage of 80 mg/m2 to 110 mg/ m2 on day 1 and 2 of up to six 4-week-dosage-cycles.

As used herein, the term "patient" preferably refers to a human in need of treatment with an afucosylated anti-CD20 antibody (e.g. a patient suffering from CD20 expressing cancer) for any purpose, and more preferably a human in need of such a treatment to treat cancer, or a precancerous condition or lesion. However, the term "patient" can also refer to non-human animals, preferably mammals such as dogs, cats, horses, cows, pigs, sheep and non-human primates, among others.

The invention further comprises an afucosylated anti-CD20 antibody with an amount of fucose is 60 % or less said antibody comprising an amino acid sequence of the variable region of the heavy chain (VH) of SEQ ID NO: 7, and an amino acid sequences of the variable region of the light chain (VL) of SEQ ID NO: 20, for use in the the treatment of a CD20 expressing cancer in combination with bendamustine.

The invention further comprises an afucosylated anti-CD20 antibody with an amount of fucose is 60 % or less, said antibody comprising an amino acid sequence of the variable region of the heavy chain (VH) of SEQ ID NO: 7, and an amino acid sequences of the variable region of the light chain (VL) of SEQ ID NO: 20, and bendamustine for use in the treatment of a CD20 expressing cancer.

Preferably the CD20 expressing cancer is a B-Cell Non-Hodgkin's lymphoma (NHL).

Preferably said humanized B-Ly1 antibody is administered in a dosage of 800 to 1200 mg on day 1, 8, 15 of a 6-week-dosage-cycle and then in a dosage of 800 to 1200 mg on day 1 of up to five 4-week-dosage-cycles, and bendamustine is administered in a dosage of 80mg/m2 to 110 mg/ m2 on day 1 and 2 of up to six 4-week-dosage-cycles.

The following examples and figures are provided to aid the understanding of the present invention, the true scope of which is set forth in the appended claims.

**Sequence Listing**

| | |
|---|---|
| **SEQ ID NO: 1** | amino acid sequence of variable region of the heavy chain (VH) of murine monoclonal anti-CD20 antibody B-Ly1. |
| **SEQ ID NO: 2** | amino acid sequence of variable region of the light chain (VL) of murine monoclonal anti-CD20 antibody B-Ly1. |
| **SEQ ID NO: 3-19** | amino acid sequences of variable region of the heavy chain (VH) of humanized B-Ly1 antibodies (B-HH2 to B-HH9, B-HL8, and B-HL10 to B-HL17) |
| **SEQ ID NO: 20** | amino acid sequences of variable region of the light chain (VL) of humanized B-Ly1 antibody B-KV1 |

### Description of the Figures

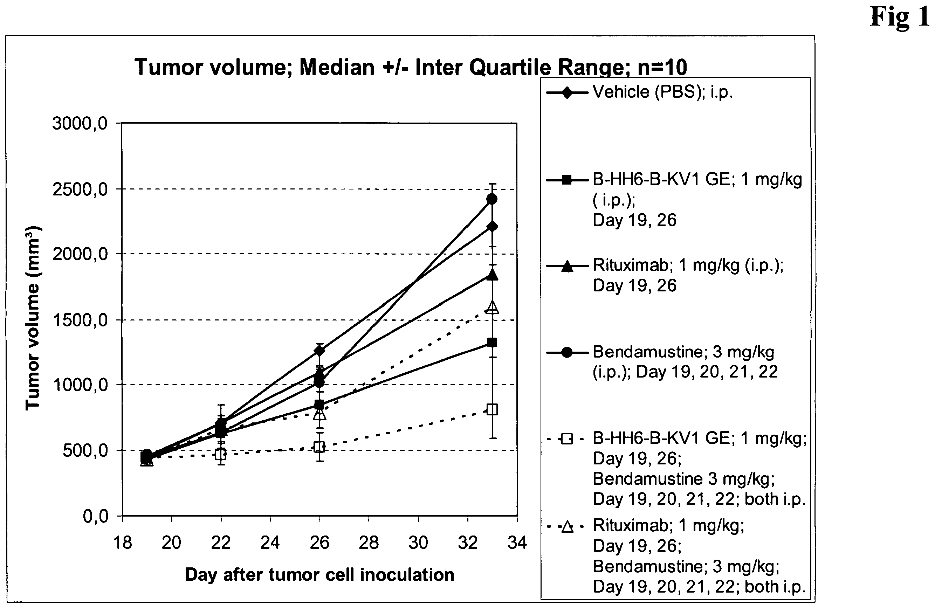

- **Figure 1**: In vivo antitumor activity of combined treatment of an afucosylated type II anti-CD20 antibody (B-HH6-B-KV1 GE) with bendamustine (in comparison with combination of rituximab (focusylated type I anti-CD20 antibody) with bendamustine and in comparison with the respective monotherapies.

### Experimental Procedures

### Experimental Procedures

### Antitumor activity of combined treatment of a type II anti-CD20 antibody (B-HH6-B-KV1 GE) with bendamustine

### Test agents

Afucosylated anti-CD20 antibody B-HH6-B-KV1 GE (afucosylated humanized B-Ly1, glycoengineered B-HH6-B-KV1, see WO 2005/044859 and WO 2007/031875) was provided as stock solution (9.4 mg/ml) from GlycArt, Schlieren, Switzerland. Antibody buffer included histidine, trehalose and polysorbate 20. Antibody solution was diluted appropriately in PBS from stock for prior injections.

Clinical grade Rituximab (Mabthera) was obtained from Hoffmann La Roche, Basel.

Bendamustine (Ribomustin®) was purchased from Mundipharma GmbH, Limburg an der Lahn, Germany. Required dilutions were adjusted from the manufactured stock solution of 2.5 mg/ml.

### Cell lines and culture conditions

The human Z138 mantle cell lymphoma cell line was routinely cultured in DMEM supplemented with 10 % fetal bovine serum (PAA Laboratories, Austria) and 2 mM L-glutamine at 37 °C in a water-saturated atmosphere at 8 % CO2. Passage 4 was used for transplantation. Cells were co-injected with Matrigel.

### Animals

Female SCID beige mice; age 3-4 weeks at arrival (purchased from Charles River, Sulzfeld, Germany) were maintained under specific-pathogen-free condition with daily cycles of 12 h light /12 h darkness according to committed guidelines (GV-Solas; Felasa; TierschG). Experimental study protocol was reviewed and approved by local government. After arrival animals were maintained in the quarantine part of the animal facility for one week to get accustomed to new environment and for observation. Continuous health monitoring was carried out on regular basis. Diet food (Provimi Kliba 3337) and water (acidified pH 2.5-3) were provided ad libitum.

### Monitoring

Animals were controlled daily for clinical symptoms and detection of adverse effects. For monitoring throughout the experiment body weight of animals was documented two times weekly and tumor volume was measured by caliper after staging.

### Treatment of animals

Animal treatment started at the day of randomisation 19 days after tumor cell inoculation. Humanized afucosylated anti-CD20 antibody B-HH6-B-KV GE or Rituximab were administered as single agents i.p. q7d on study day 19 and 26 at the indicated dosage of 1mg/kg. The corresponding vehicle was administered on the same days. Bendamustine was given i.p. on day 19, 20, 21, and 22 at 3 mg/kg. In the combination therapy groups, the chemotherapeutic agent was administered 8 hours after both antibodies on day 19.

### Tumor growth inhibition study in vivo

At the end of the experiment on day 33 after tumor cell inoculation, there was a tumor growth inhibition (TGI) as given in Table 1 in the animals given rituximab, anti-CD20 antibody B-HH6-B-KV1 GE, combination of rituximab and bendamustine or combination of anti-CD20 antibody and bendamustine, respectively, compared to the control group. Treatment with bendamustine alone did not show any antitumor activity in the present experiment.

There was more than an additive effect by comparing the anti-CD20 antibody B-HH6-B-KV1 GE/bendamustine combination group with the treatment of anti-CD20 antibody B-HH6-B-KV1 GE alone.

### Statistical evaluation

The study was terminated on day 33. Statistical evaluation was based on sAUC. Tumor growth was statistically significantly inhibited in group 2 (B-HH6-B-KV1 GE, 1 mg/kg, once weekly, i.p.) indicated by a TCR of 0.72 (CI 0.59-0.86), in group 3 (Rituximab, 1 mg/kg, once weekly, i.p.) indicated by a TCR of 0.78 (CI 0.65-0.93), in group 5 (B-HH6-B-KV1 GE, 1 mg/kg, once weekly, i.p. in combination with Bendamustine, 3 mg/kg, study days 19-22, i.p.) indicated by a TCR of 0.46 (CI 0.34-0.59) and in group 6 (Rituximab, 1 mg/kg, once weekly, i.p. in combination with Bendamustine, 3 mg/kg, study days 19-22, i.p.) indicated by a TCR of 0.67 (CI 0.54-0.81) compared to the vehicle group (group 1). Group 4 (Bendamustine, 3 mg/kg, study days 19-22, i.p.) showed a comparable tumor growth as the control group at the end of the study.

The combination of B-HH6-B-KV1 GE with Bendamustine showed a more than additive (synergistic) and statistically significant effect on tumor growth inhibition compared to the single treatments analyzed by Tukey-Kramer test (p<0.001) (see Table 1 and Figure 1)

**Table 1: Parametric TCR with low and high Confidence Interval (CI) based on sAUC and TGI (%)**

| **Group** | **Treatment schedule** | **TCR** | **95 % CI vs. group 1** | **TGI (%)** |
|---|---|---|---|---|
| 2 | B-HH6-B-KV1 GE (once weekly, 1 mg/kg) | 0.72 | [0.59-0.86] | 47 |
| 3 | Rituximab (once weekly, 1 mg/kg) | 0.78 | [0.65-0.93] | 29 |
| 4 | Bendamustine study days 19-22, 3 mg/kg | 0.9 | [0.76-1.06] | 0 |
| 5 | B-HH6-B-KV1 GE (once weekly, 1 mg/kg) + Bendamustine (study days 19-22, 3 mg/kg) | 0.46 | [0.34-0.59] | 72 |
| 6 | Rituximab (once weekly, 1 mg/kg) + Bendamustine (study days 19-22, 3 mg/kg) | 0.67 | [0.54-0.81] | 42 |

## Claims

1. Use of an afucosylated anti-CD20 antibody with an amount of fucose of 60 % or less of the total amount of oligosaccharides (sugars) at Asn297, for the manufacture of a medicament for the treatment of cancer in combination with bendamustine,
**characterized in that** said cancer is a CD20 expressing cancer and
**in that** said antibody comprises an amino acid sequence of the variable region of the heavy chain (VH) of SEQ ID NO: 7,
and an amino acid sequences of the variable region of the light chain (VL) of SEQ ID NO: 20.

2. Use according to claim 1 **characterized in that** the amount of fucose is between 40 % and 60 %.

3. Use according to any one of claims 1 or 2 **characterized in that** said CD20 expressing cancer is a B-Cell Non-Hodgkin's lymphoma (NHL).

4. Use according to any one of claims 1 to 3, **characterized in that** one or more additional other cytotoxic, chemotherapeutic or anti-cancer agents, or compounds or ionizing radiation that enhance the effects of such agents are administered.

5. Use according to any one of claims 1 to 4, **characterized in that** said antibody is administered in a dosage of 800 to 1200 mg on day 1, 8, 15 of a 6-week-dosage-cycle and then in a dosage of 800 to 1200 mg on day 1 of up to five 4-week-dosage-cycles, and bendamustine is administered in a dosage of 80 mg/m2 to 110 mg/ m2 on day 1 and 2 of up to six 4-week-dosage-cycles.

6. An afucosylated anti-CD20 antibody with an amount of fucose of 60 % or less of the total amount of oligosaccharides (sugars) at Asn297, for use in the treatment of cancer in combination with bendamustine, **characterized in that** said cancer is a CD20 expressing cancer and **in that** said antibody comprises an amino acid sequence of the variable region of the heavy chain (VH) of SEQ ID NO: 7, and an amino acid sequences of the variable region of the light chain (VL) of SEQ ID NO: 20.

7. The antibody according to the use of claim 6 **characterized in that** the amount of fucose is between 40 % and 60 %.

8. The antibody according to the use of any one of claims 6 or 7 **characterized in that** said CD20 expressing cancer is a B-Cell Non-Hodgkin's lymphoma (NHL).

9. The antibody according to the use of any one of claims 6 to 8, **characterized in that** one or more additional other cytotoxic, chemotherapeutic or anti-cancer agents, or compounds or ionizing radiation that enhance the effects of such agents are administered.

10. The antibody according to the use of any one of claims 6 to 9, **characterized in that** said antibody is administered in a dosage of 800 to 1200 mg on day 1, 8, 15 of a 6-week-dosage-cycle and then in a dosage of 800 to 1200 mg on day 1 of up to five 4-week-dosage-cycles, and bendamustine is administered in a dosage of 80 mg/m2 to 110 mg/ m2 on day 1 and 2 of up to six 4-week-dosage-cycles.

## Patentansprüche

1. Verwendung eines afucosylierten Anti-CD20-Antikörpers mit einer Menge an Fucose von 60 % oder weniger der Gesamtmenge an Oligosacchariden (Zuckern) bei Asn297 zur Herstellung eines Medikaments zur Behandlung von Krebs in Kombination mit Bendamustin, **dadurch gekennzeichnet, dass** der Krebs ein CD20 exprimierender Krebs ist und dass der Antikörper eine Aminosäuresequenz der variablen Region der schweren Kette (VH) mit der Seq.-ID Nr. 7 und eine Aminosäuresequenz der variablen Region der leichten Kette (VL) mit der Seq.-ID Nr. 20 umfasst.

2. Verwendung nach Anspruch 1, **dadurch gekennzeichnet, dass** die Menge an Fucose zwischen 40 % und 60 % liegt.

3. Verwendung nach einem der Ansprüche 1 oder 2, **dadurch gekennzeichnet, dass** der CD20 exprimierende Krebs ein B-Zell-Non-Hodgkin-Lymphom (NHL) ist.

4. Verwendung nach einem der Ansprüche 1 bis 3, **dadurch gekennzeichnet, dass** ein oder mehrere zusätzliche andere zytotoxische, chemotherapeutische oder Antikrebsmittel oder Verbindungen oder ionisierende Strahlung, welche die Wirkungen solcher Mittel verstärken, verabreicht werden.

5. Verwendung nach einem der Ansprüche 1 bis 4, **dadurch gekennzeichnet, dass** der Antikörper in einer Dosis von 800 bis 1200 mg am Tag 1, 8, 15 eines 6-wöchigen Dosierungszyklus und dann in einer Dosis von 800 bis 1200 mg am Tag 1 von bis zu fünf 4-wöchigen Dosierungszyklen verabreicht wird und Bendamustin in einer Dosis von 80 mg/m2 bis 110 mg/m2 am Tag 1 und 2 von bis zu sechs 4-wöchigen Dosierungszyklen verabreicht wird.

6. Afucosylierter Anti-CD20-Antikörper mit einer Menge an Fucose von 60 % oder weniger der Gesamtmenge an Oligosacchariden (Zuckern) bei Asn297 zur Verwendung bei der Behandlung von Krebs in Kombination mit Bendamustin, **dadurch gekennzeichnet, dass** der Krebs ein CD20 exprimierender Krebs ist und dass der Antikörper eine Aminosäuresequenz der variablen Region der schweren Kette (VH) mit der Seq.-ID Nr. 7 und eine Aminosäuresequenz der variablen Region der leichten Kette (VL) mit der Seq.-ID Nr. 20 umfasst.

7. Antikörper gemäß der Verwendung nach Anspruch 6, **dadurch gekennzeichnet, dass** die Menge an Fucose zwischen 40 % und 60 % liegt.

8. Antikörper gemäß der Verwendung nach einem der Ansprüche 6 oder 7, **dadurch gekennzeichnet, dass** der CD20 exprimierende Krebs ein B-Zell-Non-Hodgkin-Lymphom (NHL) ist.

9. Antikörper gemäß der Verwendung nach einem der Ansprüche 6 bis 8, **dadurch gekennzeichnet, dass** ein oder mehrere zusätzliche andere zytotoxische, chemotherapeutische oder Antikrebsmittel oder Verbindungen oder ionisierende Strahlung, welche die Wirkungen solcher Mittel verstärken, verabreicht werden.

10. Antikörper gemäß der Verwendung nach einem der Ansprüche 6 bis 9, **dadurch gekennzeichnet, dass** der Antikörper in einer Dosis von 800 bis 1200 mg am Tag 1, 8, 15 eines 6-wöchigen Dosierungszyklus und dann in einer Dosis von 800 bis 1200 mg am Tag 1 von bis zu fünf 4-wöchigen Dosierungszyklen verabreicht wird und Bendamustin in einer Dosis von 80 mg/m2 bis 110 mg/m2 am Tag 1 und 2 von bis zu sechs 4-wöchigen Dosierungszyklen verabreicht wird.

## Revendications

1. Utilisation d'un anticorps anti-CD20 afucosylé ayant une quantité de fucose représentant 60 % ou moins de la quantité totale d'oligosaccharides (sucres) sur l'Asn297, pour la fabrication d'un médicament destiné au traitement du cancer en association avec la bendamustine, **caractérisée en ce que** ledit cancer est un cancer exprimant CD20 et **en ce que** ledit anticorps comprend une séquence d'acides aminés de la région variable de la chaîne lourde (VH) de SEQ ID NO : 7 et une séquence d'acides aminés de la région variable de la chaîne légère (VL) de SEQ ID NO : 20.

2. Utilisation selon la revendication 1, **caractérisée en ce que** la quantité de fucose est comprise entre 40 % et 60 %.

3. Utilisation selon l'une quelconque des revendications 1 ou 2, **caractérisée en ce que** ledit cancer exprimant CD20 est un lymphome non hodgkinien à cellules B (LNH).

4. Utilisation selon l'une quelconque des revendications 1 à 3, **caractérisée en ce qu'**un ou plusieurs autres agents cytotoxiques, chimiothérapeutiques ou anticancéreux, ou des composés ou un rayonnement ionisant majorant les effets de ces agents sont administrés.

5. Utilisation selon l'une quelconque des revendications 1 à 4, **caractérisée en ce que** ledit anticorps est administré à une dose de 800 à 1200 mg aux jours 1, 8, 15 d'un cycle de dosage de 6 semaines, puis à une dose de 800 à 1200 mg au jour 1 de cinq cycles de dosage de 4 semaines au maximum et **en ce que** la bendamustine est administrée à une dose de 80 mg/m2 à 110 mg/m2 aux jours 1 et 2 de six cycles de dosage de 4 semaines au maximum.

6. Anticorps anti-CD20 afucosylé ayant une quantité de fucose représentant 60 % ou moins de la quantité totale d'oligosaccharides (sucres) sur l'Asn297, destiné à être utilisé dans le traitement du cancer en association avec la bendamustine, **caractérisé en ce que** ledit cancer est un cancer exprimant CD20 et **en ce que** ledit anticorps comprend une séquence d'acides aminés de la région variable de la chaîne lourde (VH) de SEQ ID NO : 7 et une séquence d'acides aminés de la région variable de la chaîne légère (VL) de SEQ ID NO : 20.

7. Anticorps selon l'utilisation définie dans la revendication 6, **caractérisé en ce que** la quantité de fucose est comprise entre 40 % et 60 %.

8. Anticorps selon l'utilisation définie dans l'une quelconque des revendications 6 ou 7, **caractérisé en ce que** ledit cancer exprimant CD20 est un lymphome non hodgkinien à cellules B (LNH).

9. Anticorps selon l'utilisation définie dans l'une quelconque des revendications 6 à 8, **caractérisé en ce qu'**un ou plusieurs autres agents cytotoxiques, chimiothérapeutiques ou anticancéreux, ou des composés ou un rayonnement ionisant majorant les effets de ces agents sont administrés.

10. Anticorps selon l'utilisation définie dans l'une quelconque des revendications 6 à 9, **caractérisé en ce que** ledit anticorps est administré à une dose de 800 à 1200 mg aux jours 1, 8, 15 d'un cycle de dosage de 6 semaines, puis à une dose de 800 à 1200 mg au jour 1 de cinq cycles de dosage de 4 semaines au maximum et la bendamustine est administrée à une dose de 80 mg/m2 à 110 mg/m2 aux jours 1 et 2 de six cycles de dosage de 4 semaines au maximum.


### SEQUENCE LISTING

<110> Roche Glycart AG
<120> Combination therapy of an afucosylated CD20 antibody with bendamustine
<130> 26255
<150> EP 09010489.4
   <151> 2009-08-14
<160> 27
<170> PatentIn version 3.5
<210> 1
   <211> 112
   <212> PRT
   <213> Mus sp.
<220>
   <221> MISC_FEATURE
   <223> amino acid sequence of variable region of the heavy chain (VH) of murine monoclonal anti-CD20 antibody B-Ly1
<400> 1
   Gly Pro Glu Leu Val Lys Pro Gly Ala Ser Val Lys Ile Ser Cys Lys
   1               5                   10                  15
   Ala Ser Gly Tyr Ala Phe Ser Tyr Ser Trp Met Asn Trp Val Lys Leu
               20                  25                  30
   Arg Pro Gly Gln Gly Leu Glu Trp Ile Gly Arg Ile Phe Pro Gly Asp
           35                  40                  45
   Gly Asp Thr Asp Tyr Asn Gly Lys Phe Lys Gly Lys Ala Thr Leu Thr
       50                  55                  60
   Ala Asp Lys Ser Ser Asn Thr Ala Tyr Met Gln Leu Thr Ser Leu Thr
   65                  70                  75                  80
   Ser Val Asp Ser Ala Val Tyr Leu Cys Ala Arg Asn Val Phe Asp Gly
                   85                  90                  95
   Tyr Trp Leu Val Tyr Trp Gly Gln Gly Thr Leu Val Thr Val Ser Ala
               100                 105                 110
<210> 2
   <211> 103
   <212> PRT
   <213> Mus sp.
<220>
   <221> MISC FEATURE
   <223> amino acid sequence of variable region of the light chain (VL) of murine monoclonal anti-CD20 antibody B-Ly1
<400> 2
   Asn Pro Val Thr Leu Gly Thr Ser Ala Ser Ile Ser Cys Arg Ser Ser
   1               5                   10                  15
   Lys Ser Leu Leu His Ser Asn Gly Ile Thr Tyr Leu Tyr Trp Tyr Leu
               20                  25                  30
   Gln Lys Pro Gly Gln Ser Pro Gln Leu Leu Ile Tyr Gln Met Ser Asn
           35                  40                  45
   Leu Val Ser Gly Val Pro Asp Arg Phe Ser Ser Ser Gly Ser Gly Thr
       50                  55                  60
   Asp Phe Thr Leu Arg Ile Ser Arg Val Glu Ala Glu Asp Val Gly Val
   65                  70                  75                  80
   Tyr Tyr Cys Ala Gln Asn Leu Glu Leu Pro Tyr Thr Phe Gly Gly Gly
                   85                  90                  95
   Thr Lys Leu Glu Ile Lys Arg
               100
<210> 3
   <211> 119
   <212> PRT
   <213> Artificial
<220>
   <223> amino acid sequences of variable region of the heavy chain (VH) of humanized B-Ly1 antibody (B-HH2)
<400> 3
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 4
   <211> 119
   <212> PRT
   <213> Artificial
<220>
   <223> amino acid sequences of variable region of the heavy chain (VH) of humanized B-Ly1 antibody (B-HH3)
<400> 4
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
           50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Leu Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 5
   <211> 119
   <212> PRT
   <213> Artificial
<220>
   <223> amino acid sequences of variable region of the heavy chain (VH) of humanized B-Ly1 antibody (B-HH4)
<400> 5
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
   100 105 110
   Thr Leu Val Thr Val Ser Ser
   115
<210> 6
   <211> 119
   <212> PRT
   <213> Artificial
<220>
   <223> amino acid sequences of variable region of the heavy chain (VH) of humanized B-Ly1 antibody (B-HH5)
<400> 6
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Ser Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 7
   <211> 119
   <212> PRT
   <213> Artificial
<220>
   <223> amino acid sequences of variable region of the heavy chain (VH) of humanized B-Ly1 antibody (B-HH6)
<400> 7
   Gln Val Gln Leu Val Gln Ser Gly Ala Glu Val Lys Lys Pro Gly Ser
   1 5 10 15
   Ser Val Lys Val Ser Cys Lys Ala Ser Gly Tyr Ala Phe Ser Tyr Ser
   20 25 30
   Trp Ile Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 8
   <211> 119
   <212> PRT
   <213> Artificial
<220>
   <223> amino acid sequences of variable region of the heavy chain (VH) of humanized B-Ly1 antibody (B-HH7)
<400> 8
   Gln Val Gln Leu Val Gln Ser Gly Ala Glu Val Lys Lys Pro Gly Ser
   1 5 10 15
   Ser Val Lys Val Ser Cys Lys Ala Ser Gly Tyr Ala Phe Ser Tyr Ser
   20 25 30
   Trp Ile Ser Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 9
   <211> 119
   <212> PRT
   <213> Artificial
<220>
   <223> amino acid sequences of variable region of the heavy chain (VH) of humanized B-Ly1 antibody (B-HH8)
<400> 9
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 10
   <211> 119
   <212> PRT
   <213> Artificial
<220>
   <223> amino acid sequences of variable region of the heavy chain (VH) of humanized B-Ly1 antibody (B-HH9)
<400> 10
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Ser Tyr Ser
   20 25 30
   Trp Met Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 11
   <211> 119
   <212> PRT
   <213> Artificial
<220>
   <223> amino acid sequences of variable region of the heavy chain (VH) of humanized B-Ly1 antibody (B-HL8)
<400> 11
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 12
   <211> 119
   <212> PRT
   <213> Artificial
<220>
   <223> amino acid sequences of variable region of the heavy chain (VH) of humanized B-Ly1 antibody (B-HL10)
<400> 12
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Ala Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                      80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 13
   <211> 119
   <212> PRT
   <213> Artificial
<220>
   <223> amino acid sequences of variable region of the heavy chain (VH) of humanized B-Ly1 antibody (B-HL11)
<400> 13
   Gln Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 14
   <211> 119
   <212> PRT
   <213> Artificial
<220>
   <223> amino acid sequences of variable region of the heavy chain (VH) of humanized B-Ly1 antibody (B-HL12)
<400> 14
   Glu Val Gln Leu Val Glu Ser Gly Ala Gly Leu Val Lys Pro Gly Gly
   1 5 10 15
   Ser Leu Arg Leu Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
               100                 105                 110
   Thr Leu Val Thr Val Ser Ser
           115
<210> 15
   <211> 119
   <212> PRT
   <213> Artificial
<220>
   <223> amino acid sequences of variable region of the heavy chain (VH) of humanized B-Ly1 antibody (B-HL13)
<400> 15
   Glu Val Gln Leu Val Glu Ser Gly Gly Gly Val Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 16
   <211> 119
   <212> PRT
   <213> Artificial
<220>
   <223> amino acid sequences of variable region of the heavy chain (VH) of humanized B-Ly1 antibody (B-HL14)
<400> 16
   Glu Val Gln Leu Val Glu Ser Gly Gly Gly Leu Lys Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 17
   <211> 119
   <212> PRT
   <213> Artificial
<220>
   <223> amino acid sequences of variable region of the heavy chain (VH) of humanized B-Ly1 antibody (B-HL15)
<400> 17
   Glu Val Gln Leu Val Glu Ser Gly Gly Gly Leu Val Lys Pro Gly Ser
   1               5                   10                  15
   Ser Leu Arg Leu Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 18
   <211> 119
   <212> PRT
   <213> Artificial
<220>
   <223> amino acid sequences of variable region of the heavy chain (VH) of humanized B-Ly1 antibody (B-HL16)
<400> 18
   Glu Val Gln Leu Val Glu Ser Gly Gly Gly Leu Val Lys Pro Gly Gly
   1 5 10 15
   Ser Leu Arg Val Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 19
   <211> 119
   <212> PRT
   <213> Artificial
<220>
   <223> amino acid sequences of variable region of the heavy chain (VH) of humanized B-Ly1 antibody (B-HL17)
<900> 19
<210> 20
   <211> 115
   <212> PRT
   <213> Artificial
<220>
   <223> amino acid sequences of variable region of the light chain (VL) of humanized B-Ly1 antibody B-KV1
<400> 20
   Asp Ile Val Met Thr Gln Thr Pro Leu Ser Leu Pro Val Thr Pro Gly
   1               5                   10                  15
   Glu Pro Ala Ser Ile Ser Cys Arg Ser Ser Lys Ser Leu Leu His Ser
               20                  25                  30
   Asn Gly Ile Thr Tyr Leu Tyr Trp Tyr Leu Gln Lys Pro Gly Gln Ser
           35                  40                  45
   Pro Gln Leu Leu Ile Tyr Gln Met Ser Asn Leu Val Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ala Gln Asn
                   85                  90                  95
   Leu Glu Leu Pro Tyr Thr Phe Gly Gly Gly Thr Lys Val Glu Ile Lys
               100                 105                 110
   Arg Thr Val
           115
<210> 21
   <211> 144
   <212> PRT
   <213> Homo sapiens
<400> 21
   Met Trp Leu Gln Ser Leu Leu Leu Leu Gly Thr Val Ala Cys Ser Ile
   1 5 10 15
   Ser Ala Pro Ala Arg Ser Pro Ser Pro Ser Thr Gln Pro Trp Glu His
   20 25 30
   Val Asn Ala Ile Gln Glu Ala Arg Arg Leu Leu Asn Leu Ser Arg Asp
   35 40 45
   Thr Ala Ala Glu Met Asn Glu Thr Val Glu Val Ile Ser Glu Met Phe
   50 55 60
   Asp Leu Gln Glu Pro Thr Cys Leu Gln Thr Arg Leu Glu Leu Tyr Lys
   65 70 75 80
   Gln Gly Leu Arg Gly Ser Leu Thr Lys Leu Lys Gly Pro Leu Thr Met
   85 90 95
   Met Ala Ser His Tyr Lys Gln His Cys Pro Pro Thr Pro Glu Thr Ser
   100 105 110
   Cys Ala Thr Gln Ile Ile Thr Phe Glu Ser Phe Lys Glu Asn Leu Lys
   115 120 125
   Asp Phe Leu Leu Val Ile Pro Phe Asp Cys Trp Glu Pro Val Gln Glu
   130 135 140
<210> 22
   <211> 144
   <212> PRT
   <213> Homo sapiens
<400> 22
<210> 23
   <211> 152
   <212> PRT
   <213> Homo sapiens
<400> 23
   Met Ser Arg Leu Pro Val Leu Leu Leu Leu Gln Leu Leu Val Arg Pro
   1 5 10 15
   Gly Leu Gln Ala Pro Met Thr Gln Thr Thr Pro Leu Lys Thr Ser Trp
   20 25 30
   Val Asn Cys Ser Asn Met Ile Asp Glu Ile Ile Thr His Leu Lys Gln
   35 40 45
   Pro Pro Leu Pro Leu Leu Asp Phe Asn Asn Leu Asn Gly Glu Asp Gln
   50 55 60
   Asp Ile Leu Met Glu Asn Asn Leu Arg Arg Pro Asn Leu Glu Ala Phe
   65 70 75 80
   Asn Arg Ala Val Lys Ser Leu Gln Asn Ala Ser Ala Ile Glu Ser Ile
   85 90 95
   Leu Lys Asn Leu Leu Pro Cys Leu Pro Leu Ala Thr Ala Ala Pro Thr
                       100                 105                 110
   Arg His Pro Ile His Ile Lys Asp Gly Asp Trp Asn Glu Phe Arg Arg
           115                 120                 125
   Lys Leu Thr Phe Tyr Leu Lys Thr Leu Glu Asn Ala Gln Ala Gln Gln
       130                 135                 140
   Thr Thr Leu Ser Leu Ala Ile Phe
   145                 150
<210> 24
   <211> 152
   <212> PRT
   <213> Homo sapiens
<400> 24
<210> 25
   <211> 152
   <212> PRT
   <213> Homo sapiens
<900> 25
<210> 26
   <211> 554
   <212> PRT
   <213> Homo sapiens
<400> 26
   Met Thr Ala Pro Gly Ala Ala Gly Arg Cys Pro Pro Thr Thr Trp Leu
   1               5                   10                  15
   Gly Ser Leu Leu Leu Leu Val Cys Leu Leu Ala Ser Arg Ser Ile Thr
               20                  25                  30
   Glu Glu Val Ser Glu Tyr Cys Ser His Met Ile Gly Ser Gly His Leu
           35                  40                  45
   Gln Ser Leu Gln Arg Leu Ile Asp Ser Gln Met Glu Thr Ser Cys Gln
       50                  55                  60
   Ile Thr Phe Glu Phe Val Asp Gln Glu Gln Leu Lys Asp Pro Val Cys
   65                  70                  75                  80
   Tyr Leu Lys Lys Ala Phe Leu Leu Val Gln Asp Ile Met Glu Asp Thr
                   85                  90                  95
   Met Arg Phe Arg Asp Asn Thr Pro Asn Ala Ile Ala Ile Val Gln Leu
               100                 105                 110
   Gln Glu Leu Ser Leu Arg Leu Lys Ser Cys Phe Thr Lys Asp Tyr Glu
           115                 120                 125
   Glu His Asp Lys Ala Cys Val Arg Thr Phe Tyr Glu Thr Pro Leu Gln
       130                 135                 140
   Leu Leu Glu Lys Val Lys Asn Val Phe Asn Glu Thr Lys Asn Leu Leu
   145                 150                 155                 160
   Asp Lys Asp Trp Asn Ile Phe Ser Lys Asn Cys Asn Asn Ser Phe Ala
                   165                 170                 175
   Glu Cys Ser Ser Gln Asp Val Val Thr Lys Pro Asp Cys Asn Cys Leu
               180                 185                 190
   Tyr Pro Lys Ala Ile Pro Ser Ser Asp Pro Ala Ser Val Ser Pro His
           195                 200                 205
   Gln Pro Leu Ala Pro Ser Met Ala Pro Val Ala Gly Leu Thr Trp Glu
       210                 215                 220
   Asp Ser Glu Gly Thr Glu Gly Ser Ser Leu Leu Pro Gly Glu Gln Pro
   225                 230                 235                 240
   Leu His Thr Val Asp Pro Gly Ser Ala Lys Gln Arg Pro Pro Arg Ser
                   245                 250                 255
   Thr Cys Gln Ser Phe Glu Pro Pro Glu Thr Pro Val Val Lys Asp Ser
               260                 265                 270
   Thr Ile Gly Gly Ser Pro Gln Pro Arg Pro Ser Val Gly Ala Phe Asn
           275                 280                 285
   Pro Gly Met Glu Asp Ile Leu Asp Ser Ala Met Gly Thr Asn Trp Val
       290                 295                 300
   Pro Glu Glu Ala Ser Gly Glu Ala Ser Glu Ile Pro Val Pro Gln Gly
   305                 310                 315                 320
   Thr Glu Leu Ser Pro Ser Arg Pro Gly Gly Gly Ser Met Gln Thr Glu
                   325                 330                 335
   Pro Ala Arg Pro Ser Asn Phe Leu Ser Ala Ser Ser Pro Leu Pro Ala
               340                 345                 350
   Ser Ala Lys Gly Gln Gln Pro Ala Asp Val Thr Gly Thr Ala Leu Pro
           355                 360                 365
   Arg Val Gly Pro Val Arg Pro Thr Gly Gln Asp Trp Asn His Thr Pro
       370                 375                 380
   Gln Lys Thr Asp His Pro Ser Ala Leu Leu Arg Asp Pro Pro Glu Pro
   385                 390                 395                 400
   Gly Ser Pro Arg Ile Ser Ser Leu Arg Pro Gln Gly Leu Ser Asn Pro
                   405                 410                 415
   Ser Thr Leu Ser Ala Gln Pro Gln Leu Ser Arg Ser His Ser Ser Gly
               420                 425                 430
   Ser Val Leu Pro Leu Gly Glu Leu Glu Gly Arg Arg Ser Thr Arg Asp
           435                 440                 445
   Arg Arg Ser Pro Ala Glu Pro Glu Gly Gly Pro Ala Ser Glu Gly Ala
       450                 455                 460
   Ala Arg Pro Leu Pro Arg Phe Asn Ser Val Pro Leu Thr Asp Thr Gly
   465                 470                 475                 480
   His Glu Arg Gln Ser Glu Gly Ser Ser Ser Pro Gln Leu Gln Glu Ser
                   485                 490                 495
   Val Phe His Leu Leu Val Pro Ser Val Ile Leu Val Leu Leu Ala Val
               500                 505                 510
   Gly Gly Leu Leu Phe Tyr Arg Trp Arg Arg Arg Ser His Gln Glu Pro
           515                 520                 525
   Gln Arg Ala Asp Ser Pro Leu Glu Gln Pro Glu Gly Ser Pro Leu Thr
       530                 535                 540
   Gln Asp Asp Arg Gln Val Glu Leu Pro Val
   545                 550
<210> 27
   <211> 554
   <212> PRT
   <213> Homo sapiens
<400> 27
   Met Thr Ala Pro Gly Ala Ala Gly Arg Cys Pro Pro Thr Thr Trp Leu
   1               5                   10                  15
   Gly Ser Leu Leu Leu Leu Val Cys Leu Leu Ala Ser Arg Ser Ile Thr
               20                  25                  30
   Glu Glu Val Ser Glu Tyr Cys Ser His Met Ile Gly Ser Gly His Leu
           35                  40                  45
   Gln Ser Leu Gln Arg Leu Ile Asp Ser Gln Met Glu Thr Ser Cys Gln
       50                  55                  60
   Ile Thr Phe Glu Phe Val Asp Gln Glu Gln Leu Lys Asp Pro Val Cys
   65                  70                  75                  80
   Tyr Leu Lys Lys Ala Phe Leu Leu Val Gln Asp Ile Met Glu Asp Thr
                   85                  90                  95
   Met Arg Phe Arg Asp Asn Thr Pro Asn Ala Ile Ala Ile Val Gln Leu
               100                 105                 110
   Gln Glu Leu Ser Leu Arg Leu Lys Ser Cys Phe Thr Lys Asp Tyr Glu
           115                 120                 125
   Glu His Asp Lys Ala Cys Val Arg Thr Phe Tyr Glu Thr Pro Leu Gln
       130                 135                 140
   Leu Leu Glu Lys Val Lys Asn Val Phe Asn Glu Thr Lys Asn Leu Leu
   145                 150                 155                 160
   Asp Lys Asp Trp Asn Ile Phe Ser Lys Asn Cys Asn Asn Ser Phe Ala
                   165                 170                 175
   Glu Cys Ser Ser Gln Asp Val Val Thr Lys Pro Asp Cys Asn Cys Leu
               180                 185                 190
   Tyr Pro Lys Ala Ile Pro Ser Ser Asp Pro Ala Ser Val Ser Pro His
           195                 200                 205
   Gln Pro Leu Ala Pro Ser Met Ala Pro Val Ala Gly Leu Thr Trp Glu
       210                 215                 220
   Asp Ser Glu Gly Thr Glu Gly Ser Ser Leu Leu Pro Gly Glu Gln Pro
   225                 230                 235                 240
   Leu His Thr Val Asp Pro Gly Ser Ala Lys Gln Arg Pro Pro Arg Ser
                   245                 250                 255
   Thr Cys Gln Ser Phe Glu Pro Pro Glu Thr Pro Val Val Lys Asp Ser
               260                 265                 270
   Thr Ile Gly Gly Ser Pro Gln Pro Arg Pro Ser Val Gly Ala Phe Asn
   275 280 285
   Pro Gly Met Glu Asp Ile Leu Asp Ser Ala Met Gly Thr Asn Trp Val
   290 295 300
   Pro Glu Glu Ala Ser Gly Glu Ala Ser Glu Ile Pro Val Pro Gln Gly
   305 310 315 320
   Thr Glu Leu Ser Pro Ser Arg Pro Gly Gly Gly Ser Met Gln Thr Glu
   325 330 335
   Pro Ala Arg Pro Ser Asn Phe Leu Ser Ala Ser Ser Pro Leu Pro Ala
   340 345 350
   Ser Ala Lys Gly Gln Gln Pro Ala Asp Val Thr Gly Thr Ala Leu Pro
   355 360 365
   Arg Val Gly Pro Val Arg Pro Thr Gly Gln Asp Trp Asn His Thr Pro
   370 375 380
   Gln Lys Thr Asp His Pro Ser Ala Leu Leu Arg Asp Pro Pro Glu Pro
   385 390 395 400
   Gly Ser Pro Arg Ile Ser Ser Leu Arg Pro Gln Gly Leu Ser Asn Pro
   405 410 415
   Ser Thr Leu Ser Ala Gln Pro Gln Leu Ser Arg Ser His Ser Ser Gly
   420 425 430
   Ser Val Leu Pro Leu Gly Glu Leu Glu Gly Arg Arg Ser Thr Arg Asp
   435 440 445
   Arg Arg Ser Pro Ala Glu Pro Glu Gly Gly Pro Ala Ser Glu Gly Ala
   450 455 460
   Ala Arg Pro Leu Pro Arg Phe Asn Ser Val Pro Leu Thr Asp Thr Gly
   465 470 475 480
   His Glu Arg Gln Ser Glu Gly Ser Phe Ser Pro Gln Leu Gln Glu Ser
   485 490 495
   Val Phe His Leu Leu Val Pro Ser Val Ile Leu Val Leu Leu Ala Val
                   500                 505                 510
   Gly Gly Leu Leu Phe Tyr Arg Trp Arg Arg Arg Ser His Gln Glu Pro
           515                 520                 525
   Gln Arg Ala Asp Ser Pro Leu Glu Gln Pro Glu Gly Ser Pro Leu Thr
       530                 535                 540
   Gln Asp Asp Arg Gln Val Glu Leu Pro Val
   545                 550